**Europäisches Patentamt**

(19) **European Patent Office**

**Office européen des brevets**

(11) Veröffentlichungsnummer: **0 014 367**
**B1**

(12) ## EUROPÄISCHE PATENTSCHRIFT

(45) Veröffentlichungstag der Patentschrift:
16.09.81

(51) Int. Cl.³: **C 07 C 50/34**

(21) Anmeldenummer: **80100276.7**

(22) Anmeldetag: **21.01.80**

(54) **Verfahren zur Herstellung von Dimethoxyanthrachinonen.**

(30) Priorität: **01.02.79 DE 2903851**

(43) Veröffentlichungstag der Anmeldung:
**20.08.80 Patentblatt 80/17**

(45) Bekanntmachung des Hinweises auf die Patenterteilung:
**16.09.81 Patentblatt 81/37**

(84) Benannte Vertragsstaaten:
**CH DE FR GB**

(56) Entgegenhaltungen:
**DE-A-2 152 991**

(73) Patentinhaber: **BAYER AG, Zentralbereich Patente, Marken und Lizenzen, D-5090 Leverkusen 1, Bayerwerk (DE)**

(72) Erfinder: **Seidler, Helmut, Dr., im Holz 26a, D-5060 Bergisch-Gladbach 3 (DE)**
Erfinder: **Gehrke, Günter, Dr., Leopold-Gmelin-Strasse 26, D-5000 Köln 80 (DE)**

Verfahren zur Herstellung von Dimethoxyanthrachinonen

Die vorliegende Erfindung bezieht sich auf ein Verfahren zur Herstellung von Dimethoxyanthrachinonen durch Umsetzung von Dinitroanthrachinonen mit Methanol in Gegenwart von Kaliumhydroxid.

In der Literatur werden bereits mehrere Verfahren zur Herstellung von Dimethoxyanthrachinonen aus entsprechenden Dinitroanthrachinonen beschrieben.

In der Deutschen Patentschrift 77 818 werden Mischungen von Dinitroanthrachinonen, welche durch Nitrierung von Anthrachinon mit Salpetersäure-/Schwefelsäure-Mischungen erhalten werden, in methanolischer Suspension mit Alkalimetall- oder Erdalkalimethallhydroxiden umgesetzt. Dabei erhält man jedoch ca. 30–40% Nebenprodukte, wie Amino- und Hydroxyanthrachinone u.a. (vgl. dazu entsprechende Angaben in DOS 2 607 036, S.2).

In der Deutschen Offenlegungsschrift 2 152 991 wird ein Verfahren zur Herstellung von 1,5- und 1,8-Dimethoxyanthrachinonen oder deren Gemischen beschrieben, das darin besteht, dass man 1,5- und/oder 1,8-Dinitroanthrachinone mit Kaliumhydroxid in Methanol erhitzt. In der DOS 2 607 036, S. 2, wird festgestellt, dass auch bei diesem Verfahren die Nebenreaktion nicht in ausreichendem Masse verhindert werden, so dass immer noch 10–20 Gew.-% an Nebenprodukten entstehen. Darüber hinaus besitzt das Verfahren den Nachteil, dass lange Reaktionszeiten zwischen 18 und 50 h zur vollständigen Umsetzung benötigt werden. Nachteilig ist auch die angewandte Aufarbeitungsmethode, das Verdünnen mit Wasser und anschliessende Filtration. Die dabei anfallenden Methanol-Wasser-Gemische können nicht ohne kostspielige Aufarbeitungsverfahren ins Abwasser gegeben werden.

Ein weiteres Verfahren zur Herstellung von $\alpha$-Methyläthern des Anthrachinons wird in der DOS 2 314 696 beschrieben, in welcher $\alpha$-Nitroanthrachinone mit Methanol im Überschuss in Gegenwart von Kaliumcarbonat umgesetzt werden. Als Kriterium für die Qualität der Reaktionsprodukte unter diesen Bedingungen wird ausschliesslich der $N_2$-Gehalt der Produkte angeführt, während genau analytische Daten über die Zusammensetzung der Produkte fehlen. In der DOS 2 607 036, S. 2 wird dementsprechend folgerichtig dargelegt, dass die in DOS 2 314 696 angegebenen $N_2$-Gehalte der Reaktionsprodukte von weniger als 0,5 % Gehalten von etwa 10% an Nebenprodukten (hauptsächlich Aminoanthrachinon) entsprechen.

Schliesslich wird in der DOS 2 607 036 und in der Japanischen Patentschrift 53 101 355 ein Verfahren zur Herstellung von Methoxyanthrachinonen durch Umsetzung von Nitroanthrachinonen mit Methanol und Alkali in einem Medium, das überwiegend aus Methanol besteht, beansprucht. Hierbei wird der Reaktionsmischung gasförmiger molekularer Sauerstoff zugeführt. Die Nachteile dieses Verfahrens liegen auf der Hand: Es werden Reaktionszeiten von 35 h benötigt, die nur durch Zusatz eines weiteren Lösungsmittels verringert werden können.

Die Zufuhr von Sauerstoff bedeutet ein sicherheitstechnisches Risiko, da die Gefahr einer Explosion besteht, worauf in der Patentschrift auf S. 6 selbst hingewiesen wird.

Aufgabe der vorliegenden Erfindung war es nun, ein Verfahren zur Herstellung von Dimethoxyanthrachinonen durch Umsetzung entsprechender Dinitroanthrachinone zu finden, welches die aus der vorbeschriebenen Literatur bekannten Mängel und Nachteile umgeht und unter Erzielung hoher Reinheiten und Ausbeuten wirtschaftlich und umweltschonend arbeitet. Darüber hinaus sollte das neue Verfahren kein sicherheitstechnisches Risiko in sich bergen.

Es wurde nun gefunden, dass man Dimethoxyanthrachinone oder deren Gemische in hohen Ausbeuten und in hoher Reinheit erhalten kann, wenn man die Umsetzung der Dinitroanthrachinone mit Kaliumhydroxid in Methanol so durchführt, dass man 1 Teil Dinitroanthrachinon mit 2–8 Teilen Methanol und 0,5–1,5 Teilen Kaliumhydroxid in Gegenwart von 0,05 bis 0,5 Teilen Amidosulfonsäure umsetzt, wobei «Teile» Gewichtsteile bedeuten.

Überraschenderweise zeigte es sich dabei, dass die Bildung von Nebenprodukten, wie sie hinreichend aus der vorbeschriebenen Literatur bekannt war, praktisch völlig unterdrückt werden kann, wenn der Reaktionsmischung Amidosulfonsäure zugesetzt wird. Ausserdem erwies es sich als günstig, unterhalb der Siedetemperatur des Methanols zu arbeiten; Amino- und Hydroxyanthrachinone treten unter diesen Bedingungen praktisch nicht auf.

Bei der praktischen Durchführung des Verfahrens wird vorzugsweise so vorgegangen, dass 1 Teil Dinitroanthrachinon in 3–5 Teilen Methanol mit 0,6–0,8 Teilen Kaliumhydroxid in Gegenwart von 0,1–0,3 Teilen Amidosulfonsäure umgesetzt wird.

Manchmal erweist es sich zur Erzielung eines vollständigen Umsatzes als günstig, der Reaktionsmischung 0,01 bis 0,06 Teile, vorzugsweise 0,02 bis 0,04 Teile, eines handelsüblichen Emulgators zuzusetzen.

Nach dem erfindungsgemässen Verfahren werden bevorzugt 1,5- bzw. 1,8-Dinitroanthrachinon oder deren Gemische eingesetzt. Das erfindungsgemässe Verfahren ist jedoch auch anwendbar auf technische Dinitroanthrachinongemische, wie sie bei der technischen Dinitrierung des Anthrachinons anfallen. Solche Gemische sind z.B. in der OS 2 637 733 beschrieben und werden als 1,X- oder X,Y-Dinitroanthrachinon bezeichnet, da sie auch $\alpha,\beta$- und $\beta,\beta$-Dinitroanthrachinone enthalten.

Die Reaktionstemperatur kann zwischen 50 und 90 °C liegen, wenn unter atmosphärischem Druck gearbeitet wird, wobei die Reaktion in der Regel nach 5–15 h beendet ist. Wird unter Druck gearbeitet, kann die Umsetzung auch bei höheren Temperaturen, etwa 90–150 °C, durchgeführt werden, wobei sich Drücke von 4–10 Atmosphären einstellen und die Umsetzung in der Regel nach 3–5 h beendet ist.

Besonders bevorzugt ist die Durchführung des erfindungsgemässen Verfahrens jedoch bei Temperaturen von 60–70 °C.

Das Reaktionsende wird zweckmässigerweise durch Chromatographie bestimmt. Dazu wird eine der Reaktionsmischung entnommene und aufgearbeitete Probe dünnschichtchromatographisch auf Nitroanthrachinone untersucht. Bei Reaktionsende liegen Restgehalte an Nitroanthrachinonen von ≪ 1 Gew.-% vor. Nebenprodukte, wie Amino- oder Hydroxyanthrachinone, sind praktisch nicht enthalten.

Zur Isolierung der Reaktionsprodukte bestehen mehrere Möglichkeiten. Am wirtschaftlichsten ist es, das im allgemeinen gut kristallisierte Reaktionsprodukt durch Filtration vom Methanol zu trennen. Das Methanolfiltrat kann sodann einer geeigneten Rektifikation zugeführt werden unter Wiedergewinnung von reinem Methanol; es kann aber auch teilweise undestilliert in die Reaktion recyclisiert werden.

Eine weitere Möglichkeit der Aufarbeitung besteht im Eindampfen der gesamten Reaktionsmischung unter Erhalt von wiederverwendbarem Methanol.

Die Rückstände aus der Filtration bzw. Eindampfung enthalten das Dimethoxyanthrachinon und darüber hinaus das bei der Reaktion gebildete Kaliumnitrit. Zur Abtrennung des Kaliumnitrits wird daher eine Nachbehandlung in wässrigem Medium mit Amidosulfonsäure angeschlossen, danach filtriert, gewaschen und getrocknet.

Eine weitere Möglichkeit der Aufarbeitung besteht darin, aus dem Rückstand der Filtration das Kaliumnitrit durch eine Wasserwäsche herauszulösen und so, nach Trocknung, direkt das hochreine Dimethoxyanthrachinon zu erhalten.

Aus den geschilderten Aufarbeitungsverfahren erhält man Dimethoxyanthrachinone mit Gehalten von ≥ 94%, nicht umgesetzte Dinitroanthrachinone liegen in Restgehalten von ≤ 1% vor, Nebenprodukte, wie Amino- und Hydroxyanthrachinone, sind praktisch nicht nachweisbar. Die Ausbeuten, bezogen auf eingesetztes Dinitroanthrachinon, liegen bei 90 bis 99% der Theorie.

Die nach dem erfindungsgemässen Verfahren hergestellten Dimethoxyanthrachinone oder deren Gemische sind wertvolle Farbstoffzwischenprodukte. Durch in der Farbstoffchemie übliche Reaktionen, wie Verseifung, Nitrierung, Reduktion und Bromierung lassen sich daraus eine Reihe technischer Farbstoffe für die Polyester- und Wollefärbung herstellen.

Das erfindungsgemässe Verfahren sei durch folgende Beispiele näher erläutert. Die Qualitätsangaben basieren auf quantitativer Säulenchromatographie.

Beispiel 1

100 Teile 1,5-Dinitroanthrachinon (Qualität 98%) werden in 160 Teilen Methanol verrührt. In die Suspension lässt man anschliessend unter Rühren eine Lösung von 70 Teilen Kaliumhydroxid in 160 Teilen Methanol einfliessen. Nach Zusatz von 10 Teilen Amidosulfonsäure wird unter Rühren ca. 13 h auf 68 °C erhitzt. Nachdem in einer Probe nachgewiesen worden ist, dass die Umsetzung vollständig ist, wird der Ansatz im Rotationsverdampfer bis zur Trockne eingeengt. Der Rückstand wird in 1000 Teilen Wasser verrührt und in eine Lösung von 70 Teilen Amidosulfonsäure in 1000 Teilen Wasser eingetragen. Nach Filtration, Wäsche mit Wasser und Trocknung erhält man 89 Teile eines 1,5-Dimethoxyanthrachinons mit 95,4% Gehalt. Der Gehalt an 1-Amino-5-methoxyanthrachinon liegt unter 0,3%. Dies entspricht einer Ausbeute von 97% der Theorie. Das bei der Destillation angefallene Methanol kann wiederverwendet werden; das bei der Amidosulfonsäurebehandlung angefallene wässrige Filtrat ist absolut schadlos.

Beispiel 2

Verfährt man wie in Beispiel 1 beschrieben und isoliert den Feststoff nach beendeter Reaktion durch Filtration, Wäsche mit Wasser und Trocknung, so erhält man 88 Teile eines gelben Feststoffes mit 99% 1,5-Dimethoxyanthrachinon. Dies entspricht einer Ausbeute von 99% der Theorie. Nitro- und Aminoanthrachinone sind praktisch nicht nachweisbar.

Die methanolische Mutterlauge wird destillativ unter Rückgewinnung des Methanols aufgearbeitet, das Waschfiltrat ist nach Zerstörung des Kaliumnitrits mit Amidosulfonsäure schadlos.

Beispiel 3

Eine Mischung aus 100 Teilen 1,5-Dinitroanthrachinon (Gehalt 98%), 320 Teilen Methanol, 70 Teilen Kaliumhydroxid und 10 Teilen Amidosulfonsäure wird unter Rühren 17 h auf 63 °C erwärmt. Nach beendeter Reaktion wird die Reaktionsmischung abgekühlt und der Feststoff durch Filtration isoliert. Der Rückstand wird mit Wasser gewaschen und getrocknet. Man erhält 88 Teile eines gelben, in gut ausgebildeten Nadeln kristallisierten Produktes mit 99% 1,5-Dimethoxyanthrachinon. Die Ausbeute beträgt somit 99% der Theorie. Nitro- und Aminoanthrachinone sind praktisch nicht nachweisbar.

Beispiel 4

Eine Mischung aus 320 Teilen Methanol, 70 Teilen Kaliumhydroxid, 100 Teilen 1,5-Dinitroanthrachinonen (Qualität 98%) und 10 Teilen Amidosulfonsäure wird 9 h zum Rückfluss erhitzt. Nach Eindampfen der Reaktionsmischung wird der Rückstand in 2000 Teilen Wasser verrührt und die erhaltene Suspension mit 70 Teilen 1:1 verdünnter HCl-konc. versetzt. Anschliessend werden 40 Teile Amidosulfonsäure nachgesetzt. Nach kurzem Verrühren wird abgesaugt, gewaschen und getrocknet unter Erhalt von 89,5 Teilen Reaktionsprodukt. Der Gehalt an 1,5-Dimethoxyanthrachinon beträgt 94,6%, was einer Ausbeute von ca. 97% der Theorie entspricht.

## Beispiel 5

Zu Vergleichszwecken wurde das Verfahren des Beispiels 4 wiederholt, wobei die Reaktion jedoch ohne Amidosulfonsäurezusatz durchgeführt wurde. Das analog erhaltene Produkt von 88 Teilen enthält nur 80% 1,5-Dimethoxyanthrachinon und grössere Mengen Nebenprodukte, so dass nur eine Ausbeute von ca. 84% der Theorie erzielt wurde.

## Beispiel 6

Zu einer gerührten Suspension von 100 Teilen 1,8-Dinitroanthrachinon (Gehalt 98%) in 160 Teilen Methanol wird eine Lösung von 70 Teilen Kaliumhydroxid in 160 Teilen Methanol getropft. Nach Zusatz von 25 Teilen Amidosulfonsäure wird unter Rühren 14 h auf 68 °C erhitzt. Nach Abkühlen auf Raumtemperatur wird filtriert, der Rückstand mit Wasser gewaschen und getrocknet. Man erhält 84 Teile eines in gut ausgebildeten Rhomben kristallisierten gelben Produktes, welches 94,1% 1,8-Dimethoxyanthrachinon enthält. Dies entspricht einer Ausbeute von 90% der Theorie.

## Beispiel 7

100 Teile einer Mischung aus 1,5-/1,8-Dinitroanthrachinon (Gehalt 58,8% 1,5-Dinitroanthrachinon und 39% 1,8-Dinitroanthrachinon) werden in 320 Teilen Methanol verrührt. Nach Zugabe von 75 Teilen Kaliumhydroxid und 18 Teilen Amidosulfonsäure wird ca. 9 h zum Rückfluss erhitzt. Nachdem in einer Probe die vollständige Umsetzung nachgewiesen wurde, wird die Reaktionsmischung im Rotationsverdampfer zur Trockne eingeengt. Der Rückstand wird in 1000 Teilen Wasser verrührt und mit soviel fester Amidosulfonsäure behandelt, dass alles Kaliumnitrit zerstört ist. Nach Filtration, Wäsche mit Wasser und Trocknung erhält man 89,5 Teile eines 1,5-/1,8-Dimethoxyanthrachinon-Gemisches mit folgender Analyse: 56,2% 1,5-Dimethoxyanthrachinon und 35,6% 1,8-Dimethoxyanthrachinon, das entspricht Ausbeuten von 95% bzw. 91% der Theorie.

## Beispiel 8

100 Teile 1,x-Dinitroanthrachinon (12,3% 1,5-3,2% 1,6-, 12,7% 1,7- und 64% 1,8-Dinitroanthrachinon) werden in eine Lösung von 50 Teilen Kaliumhydroxid in 400 Teilen Methanol eingetragen und in 2 h auf 120 °C erhitzt. Man rührt die Mischung noch 4 h bei 120 °C und 5–6 bar und destilliert anschliessend im Rotationsverdampfer zu Trockne. Der Rückstand wird in 1000 Teilen Wasser angerührt und mit ca. 40 Teilen Amidosulfonsäure versetzt, bis sich kein Nitrit mehr nachweisen lässt. Man saugt ab, wäscht mit Wasser und trocknet und erhält 85 Teile 1,x-Dimethoxyanthrachinon (81% 1,5-, 10,9% 1,6/1,7-, 65% 1,8 Dimethoxy- und 4,2% 1,5/1,8-Hydroxymethyl-anthrachinon).

## Beispiel 9

In 160 Teilen Methanol werden 3 Teile eines neutralen, handelsüblichen Emulgators gelöst. Unter Rühren werden 100 Teile 1,8-Dinitro-anthrachinon (Gehlat 98%) eingetragen. In der so erhaltenen Suspension werden anschliessend 240 Teile einer Kaliummethylatlösung gegeben, die durch Verrühren von 80 Teilen Kaliumhydroxid in 160 Teilen Methanol hergestellt wurde; die Temperatur der Reaktionsmischung beträgt dabei etwa 30 °C. Nach Zusatz von 35 Teilen Amidosulfonsäure wird die Mischung auf 66–68 °C erhitzt und unter intensivem Rühren 16 Stunden auf dieser Temperatur belassen.

Nach Abkühlen der Reaktionsmischung wird der Feststoff durch Filtration isoliert und durch Waschen mit Methanol und Wasser gereinigt. Nach Trocknung erhält man 82 Teile 1,8-Dimethoxyanthrachinon mit einem Gehalt von 99%. Nitroderivate sind praktisch nicht mehr nachweisbar. Die Ausbeute beträgt 92% der Theorie.

## Patentansprüche

1. Verfahren zur Herstellung von Dimethoxyanthrachinonen durch Umsetzung von Dinitroanthrachinonen mit Kaliumhydroxid in Methanol, dadurch gekennzeichnet, dass man 1 Gew.-Teil Dinitroanthrachinon mit 2–8 Gew.-Teilen Methanol und 0,5–1,5 Gew.-Teilen Kaliumhydroxid in Gegenwart von 0,05–0,5 Gew.-Teilen Amidosulfonsäure bis zum vollständigen Austausch der Nitrogruppen umsetzt.

2. Verfahren nach Anspruch 1, dadurch gekennzeichnet, dass man in Gegenwart von 0,1–0,3 Gew.-Teilen Amidosulfonsäure arbeitet.

## Revendications

1. Procédé de production de diméthoxyanthraquinones par réaction de dinitro-anthraquinones avec de l'hydroxyde de potassium dans du méthanol, caractérisé en ce qu'on fait réagir 1 partie en poids de dinitro-anthraquinone avec 2–8 parties en poids de méthanol et 0,5–1,5 partie en poids d'hydroxyde de potassium en présence de 0,05–0,5 partie en poids d'acide amidosulfonique jusqu'à l'échange total des groupes nitro.

2. Procédé suivant la revendication 1, caractérisé en ce qu'on opère en présence de 0,1–0,3 partie en poids d'acide amidosulfonique.

## Claims

1. Process for the preparation of dimethoxyanthraquinones by reacting dinitroanthraquinones with potassium hydroxide in methanol, characterised in that 1 part by weight of dinitroanthraquinone is reacted with 2–8 parts by weight of methanol and 0,5–1,5 parts by weight of potassium hydroxide in the presence of 0,05–0,5 part by weight of amidosulphonic acid until all the nitro groups have been replaced.

2. Process according to claim 1, characterised in that the reaction is carried out in the presence of 0,1–0,3 part by weight of amidosulphonic acid.